# EUROPEAN PATENT SPECIFICATION

(11) **EP 0 938 329 B1**
(45) Date of publication and mention of the grant of the patent: **29.05.2002**
(21) Application number: 97945086.3
(22) Date of filing: 09.10.1997
(51) Int. Cl.: A61K 38/03, C07K 14/705

(54) **IMMUNOGENIC TLP COMPOSITION**
IMMUNOGENE TLP-ZUSAMMENSETZUNG
COMPOSITION IMMUNOGENE A BASE DE PARTICULES LIBEREES PAR LA TUMEUR

(30) Priority: 09.10.1996 IT RM960687
(43) Date of publication of application: 01.09.1999
(73) Proprietor: Unihart Corporation, Dublin 2 (IE)
(72) Inventor: TARRO, Giulio, Istituto Farmacoterapico Ita. Spa, I-00197 Roma (IT)
(74) Representative: Bianchetti, Giuseppe, Prof.
(86) International application number: IT9700240
(87) International publication number: WO9815282

(56) References cited:
- EP-A- 0 283 443
- WO-A-94/01458
- TARRO, GIULIO ET AL: "Human tumor antigens inducing in vivo delayed hypersensitivity and in vitro mitogenic activity" ONCOLOGY (1983), 40(4), 248-54 CODEN: ONCOBS;ISSN: 0030-2414, XP002045904

## Description

### Background of the invention

The present invention relates to the field of immunotherapy of tumoral diseases.

### Prior art

The oncologic research has been directing its efforts for many times towards the problem of immunotherapy of tumoral diseases, on the basis of the reasonable possibility of finding a therapeutically useful solution through the manipulation of the immunooncolytic reaction that the human organism can develop spontaneously. The initial urge towards such trend of the scientific research can be recognised in the first observations (I.S. Irlin, Virology 1967 32:725; E.Klen et al. Nat.Cancer Inst.1964 32:547; G.J.Pasternak J.Nat.Cancer Inst., 1965, 34:71, S.S. Tevethia et al. Immunol. 1968, 100:358; R.Nishioka et al. Monograph 1968, 7:49)regarding the stimulation of specific humoral and cellular antibodies by the antigens of neoplastic cells both in animals and in human beings.

The immune manipulations attempted so far as an immunotherapic approach, while reflecting the knowledge successively acquired concerning the physiopathology of cancer and of the immune system of the host, on the other side also suffers from the lack of suitable immunogenic agents or from the difficulty experienced in removing the situations of block of cellular immunity which are present in the cancer patients.

As a matter of practice two main roads are originally followed:
a) the non specific activation of the host's immunity in order to strengthen the immuno-oncolytic reactions (immuno-adjuvants);
b) the specific activation of the host's immunity in order to electively stimulate the production of antibodies having oncolytic effects.

The specific approach has the advantage to direct a more specific and effective immune-response to the tumor cells, strenghthening the effects.

In any case, the lysis of tumour cells by the immune system is mediated both by direct cytotoxic mechanisms (NK cells) and by cytotoxic mechanisms that are more complex but more effective and specific, involving recognition of antigens on the surface of the tumour cells and the presence of antibodies (ADCC cytotoxicity, prevalently mediated by CD8+ cells). The immune system always operates by means of a complex network of cytokines, which modulate the action of the effector cells by means of inhibition and stimulation, with a "cascade" mechanism.

Attempts in immunotherapy have up to date been aimed at generic amplification of the cell-mediator immune response, either expanding (thymic hormones, IL-2) or activating (BCG, PPD, IFNs, IL-2, TNFs, etc.) the lymphocyte populations in a non-specific manner. Research into this effect was also through the use of a single-substance (IL'-2, IFN, etc.) which in order to induce the necessary function required to be administered at high doses, with considerable toxic effects and prohibitively high costs (Mulè JJ, Shu S., Swarz SL., Rosenberg SA., Science, 225: 1478, 1984; Hadden JW., in "Advances inimmunomodulation"., 1988, Ed. B. Bizzini and E. Bonmassar, Pitagora Press Roma).

Previous studies have shown us that combined immunotherapy (thymic hormones and a cytokine administered at a low dose, IFN-alpha or IL-2) was capable of producing a synergetic effect on the cytotoxic activities of the lymphocytes (NK, LAK, CTL activity) with respect to certain tumour cells in vitro (Favalli, C., Mastino, A., Jezzi, T., Grelli, S., Goldestein, A.L. and Garaci, E., Int. J Immunopharmacol., 11, 443-450, 1989; Mastino, A., Favalli, C., Grelli, S., Innocenti, F. and Garaci, E., Cell. Immunol., 133, 196-205, 1991). However, an increase in cytotoxic activities corresponded neither to an adequate anti-tumour response *in vivo,* nor to an increased survival rate (Favalli, C., Mastino, A., Grelli, S., Pica, F., Rasi, G., Garaci, E., Combination Therapies. pp. 275-280 Ed. by A. Goldstein and E. Garaci, Plenum Press, NY, 1992; Mastino, A., Favalli, C., Grelli, S., Rasi, G., Pica, F., Goldestein, A.L. and Garaci, E., Int. J. Cancer. 50, 493-499, 1992; Garaci, E., Pica, F., Mastino, A., Palamara, A.T., Belardelli, F. and Favalli, C., J. Immunother., 13, 7-17, 1993). On the contrary, when combined immunotherapy was preceded by chemotherapy (even using ineffective doses) there was on the other hand complete recovery from the tumour (Mastino, A., Favalli, C., Grelli, S., Rasi, G., Pica, F., Goldestein, A.L. and Garaci, E., Int. J. Cancer. 50, 493-499, 1992; Garaci, E., Pica, F., Mastino, A., Palamara, A.T., Belardelli, F. and Favalli, C., J. Immunother., 13, 7-17, 1993; Rasi, G., Sinibaldi-Vallebona P., Favalli, C., Pierimarchi, P., et al., 2nd International Symposium on Combination Therapies, documents, 1-3 May, 1992 - Santa Tecla CT).

Further studies on experimental models have shown that the immunotherapy was only effective in case of immunogenic neoplasia, and that the main role of chemotherapy (used at ineffective doses) was to render the neoplasia immunogenic (Rasi, G., Sinibaldi-Vallebona P., Favalli, C., Pierimarchi, P., et al., 2nd International Symposium on Combination Therapies, documents, 1-3 May, 1992 - Santa Tecla CT; Sinibaldi-Vallebona P., Pierimarchi, P., Ravagnan, G.P., Rasi, G., Third International Symposium on Combination Therapies, documents, 29-31 October, 1993, Houston, Texas; Sinibaldi-Vallebona P., Pierimarchi, P., Lucertini, L., Ravagnan, G.P., Rasi, G., Fourth International Symposium on Combination Therapies. 14-17 June, 1994, documents, p. 105; Rasi, G., Silecchia, G.F., Sinibaldi-Vallebona P., Pierimarchi, P., Sivilia, M., Tremiterra, S., Garaci, E., Int. J. Cancer, 57, 701-705, 1994). Recently, this strategy has also been found to be effective in treatment of solid human tumours (Rasi, G., Favalli, C., Terzoli, E., Izzo, F., Sinibaldi-Vallebona P., Pierimarchi, P., Sivilia, M., Garaci, E., Biomedicine & pharmacotherapy, 47-292, 1993). In experimental models we have thus demonstrated the low effect or lack of effect of each of the single treatments (chemotherapy, thymic hormone, cytokine), the absence of anti-tumour effect even in the presence of a considerable increase in the levels of immune activity in cells, and lysis of the tumour only in the presence of a specific cell-modulated cytotoxic activity on neoplastic cells. From these studies it can therefore be stated that the role of the antigen is decisive in order to induce a cytotoxic immune response with a significant anti-tumour effect.

When attempting to amplify the anti-neoplastic immune response the availability of antigens to induce and modulate, and the knowledge of the immunological relationships within each "target/effector" system (neoplastic cell/lymphocyte) therefore appear to be essential.

TLP complexes are protein complexes present in human tumour cells. Among these TLP proteins a protein of 240 Kda is described (Tarro G., Oncology 40, 248-253, 1983). TLP are isolated from tumour tissues as described in the European patent No. 0283443. The European patent No. 649433 identifies a TLP protein obtained from pulmonary carcinoma. The Italian patent application No. RM96A000496 indicates that TLP obtained from carcinomas of the urogenital system comprise peptides of a different sequence than those previously identified. The proteic fragments of proteins from TLP can also be produced synthetically using known methods.

In 1983 a new tumour antigen of 240 kDa was identified, extracted from the neoplastic tissue of non-small cell lung carcinoma (NSCLC) and named TLP (Tumour Liberated Particles). In the Cold Spring Harbor Laboratories (NY-USA) a structure analysis of the lung carcinoma antigen was recently performed and 100 kDa antigen was extracted, then a major TLP epitope sequence was recently identified, the polypeptide (a nonapeptide, CSH 275) was synthesised, and the relative antibody (CSH 419) was produced.

This antibody (CSH419) has proved its ability, using Western blot after immunoprecipitation and immunohystochemistry (P.A.P.), to recognise the antigen sequence in the homogenate obtained from all the neoplastic tissues (NSCLC) taken into consideration up to this point.

The peptide claimed in Seq. ID N1, with others derived from the antigenic region of TLP were described and claimed in the European Patent no. 649433 corresponding to the International Application WO 94/01458.

EP 283443 discloses a process for the extraction, from human neoplastic cells, of membrane antigens (TLP) having immunogenic properties and capable of inducing delayed hypersensitivity and specific blastization of Lymphocytes in different types of carcinoma.

Oncology 40 (1983), 248-254, discloses the immunogenic properties of an antigen extracted from TLP and its mitogenic activity on limphocyte cultures and or cancer patients.

### SUMMARY OF THE INVENTION

The present invention relates to immunogenic fragments of TLP (Tumour Liberated Particles) complex for therapeutic and immunogenic use.

The author of the present invention has now prepared full and accurate documentation of clinical use for TLP as an immuno-modulating agent (capable of stimulating the immune responses of the host) both to combat diagnosed neoplastic pathologies (immuno-therapy), and to prevent cancerous pathologies (vaccine).

On the basis of experience gained during study of experimental models, the essential requirements to enable an antigen like TLP to be used from a therapeutic point of view are the following:
1. the presence of the antigen on the surface of the tumour cells;
2. the ability to pharmacologically induce or increase expression of the antigen on the surface of the cells;
3. the ability of the antigen to stimulate lymphocytes with a specific anti-tumour activity (blastogenetic capacity and CTL (cytotoxic tymus dependent lymphocytes) activity);
4. the presence of TLP in the serum (indicates possible correlation between the serum levels and expression of the antigen on the cell surface, and the absence of systems (kidneys or other emunctory systems such as the skin or intestine) giving rapid clearance from circulation).

The positivity to these four operations are indicative of both a suitability as a therapeutic agent and as a vaccine, owing to the strong immunogenic activity demonstrated.

The object of the present invention is therefore the use of an immunogenic composition as a vaccine and as a medicament in the prevention and the treatment respectively of cancer, particularly pulmonary cancer and uro-genital cancer, comprising a least one TLP immunogenic fragment.

The immunogenic fragments of TLP protein contain at least one of the following amino acid sequences:

ArgThrAsnLysGluAlaSerIle

GlySerAlaXPheThrAsn

AsnGlnArgAsnArgAsp

Alternatively, the immunogenic composition according to the invention includes an immunogenic fragment comprising the following amino acid sequence:
GlyProProGluValClnAsnAlaAsn (Seq ID N1 of Italian patent application RM96A00496).

The object of the present invention is therefore an immunogenic composition and the use therof as a vaccine and as a medicament in the prevention and the treatment respectively of cancer, particularly pulmonary cancer and uro-genital cancer, comprising at least one protein obtained from TLP or at least an immunogenic fragment thereof.

The immunogenic phragments of TLP protein preferably contain at least one of the following amino acid sequences:

ArgThrAsnLysGluAlaSerIle (Seq ID N1)

GlySerAlaXPheThrAsn (Seq ID N2)

AsnGlnArgAsnArgAsp (Seq ID N3)

Alternatively, the immunogenic composition according to the invention includes an immunogenic fragment comprising the following amino acid sequence:
GlyProProGluValGlnAsnAlaAsn (Seq ID N1 of Italian patent application RM96A000496).

### BRIEF DESCRIPTION OF THE DRAWINGS

Figure 1 shows the result of an experiment carried out on neoplastic cells obtained from NSCLC explants using the flow cytofluorimetry technique (Facscan-BD).

The peak on the right of the histogram, reveals the binding of the anti-TLP antibodies with the correspondent antigen on the cell surface.

Figure 2 shows the result of the same experiment described in figure 1, carried out on cells from the same explants, treated with pre-immune serum as a negative control.

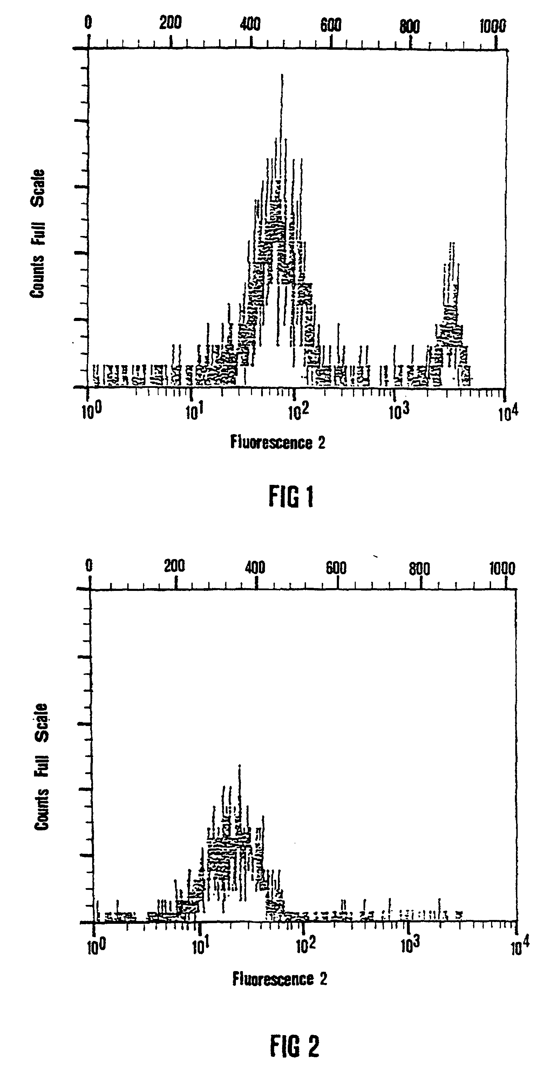

The peak shown in figure 1, comprising the values between 10³ and 10⁴ of the fluorescence, and revealing the bond between antigen and antibodies anti-TLP, is absent.

### EXPERIMENTAL DESCRIPTION

### Presence of the antigen TLP on the surface of the tumour cells.

The antigen TLP was searched for on the surface of fresh neoplastic cells obtained from non-small cell lung carcinoma explants (NSCLC) using the flow cytofluorimetry technique (Facscan-BD).

The cells were marked by addition of monoclonal anti-TLP antibody (CSH # 419, Cold Spring Harbor Lab. NJ USA) in conjunction (second step) with a second goat anti-rabbit IgG-RPE.

The TLP-antiTLP binding specificity was evaluated using non-specific antiserums or pre-immune serum. The cell phenotype specificity was evaluated by marking cells of stabilised tumour lines or fresh cells from neoplasias other than NSCLC with antiTLP.

### Ability to pharmaceutically induce or increase expression of the antigen on the surface of the cells.

The cells were processed fresh and after preparation of primary cultures (complete RPMI 1640 culture medium with the addition of FCS 10%).

Along with variation in TLP, other possible phenotype modifications (IL-2 rec (interleukin receptors), HLA-Dr (human leukocyte antigen), CD16 (lymphocyte sub-populations), etc.) induced by treatment with single or combined agents (chemotherapy, cytokines) were also studied.

In the case of TLP, freshly isolated tumor cells were obtained from surgical NSCLC patient; after 24 plating and fibroblasts separation, cells were resuspended for immunoistochemistry and cytofluorimetric assay, briefly: CSH419 antiserum was labeled with PE conjugated anti-rabbit IgG and incubated with the cells. Negative controls were obtained by the use of rabbit preimmune serum and (for immunohistochemistry also by serial dilution down to 500 fold for positive samples; no reaction was observed by staining or conjugating K562, and 2 women melanoma cell line. TLP was demonstrated on 75% of the NCLC lines studied.

Preliminary studies by confocal microscopy showed a cytoplasmatic and membrane localization of TLP by the tumor cells. TLP antigen expression has been shown to be enhanced or induced in vitro by chemotherapy treatment: primary NSCLC culture cell become TLP-positive after cisplatinum or etoposide treatment.

The freeing of TLP in the culture supernatant was checked using the ELISA test.

Simultaneously experiments consistent in administration of cis-platinum and etoposide to serum-negative NSCLC patients are carried out to test the reaction on TP production in vivo.

### Ability of Ag to stimulate lymphocytes with specific anti-tumour activity (blastogenetic ability and CTL activity).

The lymphocytes obtained from peripheral bloodstream of patients from whom the neoplastic cells were explanted (autologous lymphocytes) were marked by flow cytofluorimetry (CD4/CD25, CD8/CD25, CD56-16-3±/CD25 phenotypes) before and after treatment in vitro with TLP, both alone and in association with chemotherapy and/or cytokines.

The cytotoxic activity of the autologous lymphocytes (both treated and untreated) was determined by testing release of the ⁵¹CR after 4h, using the tumour cells obtained from explants of tumour from the same patient as target cells.

Lymphocytes from healthy individuals and from patients suffering from other neoplastic pathologies were used (as effectors) against neoplastic cell lines (sensitive and resistant NK targets) as controls to establish the specific nature of the tumour lysis.

### Presence of TLP in the serum

A test ELISA with analytical "sandwich" scheme, was carried out to determine the presence of TLP in the serum of NSCLC patients, and in serum of patient affected by other pathologies (neoplastic pathologies different from NSCLC; lung non-neoplastic pathologies) and in other controls.

### RESULTS

### Presence of the antigen TLP on the surface of the tumour cells.

The presence of TLP on lung carcinoma cells (NSCLC) was demonstrated using the method described. As an example of positive tumour, the data shown in figure 1 are reported. It is possible to note that the labeling with pre-immune serum gives no signal, while the labeling with antiTLP#419, distinctly evidences a TLP positive population.

### Ability to pharmaceutically induce or increase expression of the antigen on the surface of the cells.

Two TLP-positive tumor cell population were treated with cisplatinum and etoposide (10µg/ml) for 48 hours:
2 TLP-negative cell populations became TLP-positives after etopside treatment;
1 TLP-negative cell population became TLP-positive after cisplatinum treatment;

TLP positive cell population remained TLP positive after etoposide and cisplatinum treatment.

The results of the ELISA test used for checking the freeing of TLP in the culture supernatant are compatible to that exposed in tables II-IV, reporting the presence of TLP in the serum of NSCLC patients.

The administration of the same chemotherapics in TLP serum-negative patients, has produced an intensive positive response for TLP, checked by ELISA, after only two cycles of treatment.

### Ability of Ag to stimulate lymphocytes with specific anti-tumour activity (blastogenetic ability and CTL activity).

*In vitro* treatment of the lymphocytes of patients suffering from NSCLC with TLP induces blastic activity, in particular against certain phenotypes: activated cells that express the high affinity receptor for IL-2 (CD3+/CD25+); NK cells (CD56+/CDZ6+/CD3±); activated cytotoxic cells CD25+/CD8+.

**Table I**

| PHENOTYPES | CD25 | NK | CD25/CD8 |
|---|---|---|---|
| Untreated lymphocytes | 3.3-4.6 | 10.1-18.9 | 3.5-5.3 |
| Lymphocytes + TPL* | 5.5-16.1 | 21.4-32.2 | 8.6-11.3** |

| | | | |
|---|---|---|---|
| * µg/ml | | | |
| ** a dose-dependent effect is observed for addition of TLP to the medium and activation of the CD8+ cells (which can reach up to 20% of the entire culture population). | | | |

Treatment of the lymphocytes with TLP is also capable of inducing lytic activity of both type NK (natural killer cells, target cells K562) and CTL (on cells of its own tumour). The NK activity shows the same dose dependence seen for CD8+CD25+ cells and also appears to be closely correlated to the number of said cells. This observation, along with the absence of lytic activity (either spontaneous or TLP-induced) of a LAK type (lymphokine activated killer cells, targeting NK-resistant cells, Daudi) appears to indicate the specific nature of the activation.

The homologated lymphocytes, treated with TLP, have also shown themselves to be active on cells of a cerebral metastasis resulting from NSCLC.

### Presence of TLP in the serum

The results of the ELISA with analytical "sandwich" scheme has given the resulte exposed as follows:

**Table II**

| NSCLC | | |
|---|---|---|
| HYSTOLOGIC TYPE | N/POS. | % |
| Epidermoidal | 40/22 | 55 |
| Adenocarcinoma | 12/7 | 58 |
| **Tot.** | **52/29** | **56** |

**Table III**

| Different neoplasias from NSCLC | | |
|---|---|---|
| HYSTOLOGICAL TYPE | NEG. | % |
| SCLC | 15/15 | 100 |
| Indefinite | 7/7 | 100 |
| Carcinosarcoma | 1/1 | 100 |
| Carcinoid | 1/1 | 100 |
| Pulm. Metast. from ovary | 1/0* | |
| Melanoma | 3/3 | 100 |
| Gastric Carcinoma | 2/2 | 100 |
| **Tot.** | **30/29** | **97** |

| | | |
|---|---|---|
| * Border line | | |

**Table IV**

| Non-neoplasia lung pathologies | | |
|---|---|---|
| PATHOLOGY | N/NEG. | % |
| BOC | 21/18 | 86 |
| TBC | 2/1 | 50 |
| **Tot.** | **23/19** | **82** |

**Table V**

| Other controls | | |
|---|---|---|
| Cases | N/Neg. | % |
| z Healthy | 11/11 | 100 |
| Pregnant | 2/1 | 50 |
| **Tot.** | **13/12** | **92** |

Moreover, the measurement of the TLP levels in NSCLC patients gives a result of 56% (see table II) which must be compared with the data of 35-40% obtained from the "cyfra", the other lonely marker proposed for Lung carcinoma. Furthermore the data on the specificity of the binding indicate a specificity of 100% circa, for the TLP against a datum of 60%-70% for the "cyfra".

In a similar manner, the synthetic polypeptide (Seq ID N1) shows excellent immunogenic abilities, studies actually indicating a specific stimulation of the cytotoxic CD8 lymphocytes only in patents suffering from NSCLS neoplasia.

Experiments carried out in "skid mice" show that a vaccinal tratment with Seq. ID N1 protects the animals from a growth of 1.800.000 to 6.000.000 of tumour cells.

Analogous results are obtained for the other peptides claimed.

### FINAL CONCLUSION

The results of the studies carried out on TLP, the derivative peptides, and the peptide GlyProProGluValGlnAsnAlaAsn derived from the analogous protein extracted from urogenital-carcinoma, show an immunological anti-tumoral action directed against them.

In fact the presence of TLP on the surface of the NSCLC cells, and its demonstrated capability to stimulate lymphocytes with specific anti-tumour activity (both the blastogenetic ability and CTL activity), evidence clearly the immunogenicity of the protein. The same considerations are demonstrated to have value for the peptides derived, both Seq. ID N1 and the other two peptides Seq. ID N2 and Seq. ID N3 also derived from the antigenic region of the TLP protein.

The value of this approach is confirmed by the fact that a relevant presence of TLP in the serum of NSCLC patients was found, indicating a strict association between the presence of tumour and the level of TLP in blood. Therefore it could be suitable and effective an approach based on the administration of TLP, or the peptides thereof, as its immunogenically active sequence were demonstrated to have no homologous in other human protein.

The administration of TLP or derived peptides therefore, would be more specific and effective than an approach carried out by a non-specific activation of the host's immunity system, and less destructive than the administration of the solely chemotherapies.

Howewer the administration of the chemotherapics could also potentiate the therapeutic effects of TLP, as they have demonstrated the capability of inducing the production of the Ag both in cell cultures and in NSCLC patients originally serum-negative for TLP.

This is a direct consequence of the results of the experiment carried out with single or combined chemotherapic agents. As it was previously shown in fact, the administration of etoposide and cis-platinum in tumour cell cultures was shown to stimulate TLP production at levels compatible with those registered in the NSCLC serum-positive patients, and the administration of the same chemotherapics make the patients originally serum-negative (ELISA) for TLP, to become intensively positive after two cycles of chemotherapy.

In the case of the peptide GlyProProGluValGlnAsnAlaAsn derived from the protein analogous to TLP, extracted from urogenital-carcinoma the experiment carried out demonstrate an analogous suitabiity as immunotherapic for all the urogenital tumoral forms.

All these data are indicative of both a therapeutic and a vaccinal use owing to the strong immunogenic activity demonstrated from all these molecules. This use is suitable for humans and mammals in general for proteins obtained from TLP and analogous thereof. The same is true for the peptides illustrated in the experimental texts.

The immune response in fact can be caused both to contrast the tumour growth and expansion (treatment effective also in metastasis as it is previously shown) and to protect healthy people from developing the disease. The data obtained in "skid mice" previously shown particularly support these statements.

## Claims

1. The use of an immunogenic fragment of TLP including at least one of the amino acid sequences selected from the group comprising: ArgThrAsnLysGluAlaSerIle; GlySerAlaXPheThrAsn; AsnGlnArgAsnArgAsp, for the manufacture of a vaccine for a preventive treatment of lung cancer in mammals effective to produce in said mammal an immune response against said lung cancer.

2. The use of an immunogenic fragment of TLP including at least one of the amino acid sequences selected from the group comprising: ArgThrAsnLysGluAlaSerIle; GlySerAlaXPheThrAsn; AsnGlnArgAsnArgAsp, for the manufacture of an immunogenic medicament for an active specific immunotherapic treatment of lung cancer in a mammal, effective to produce in said mammal an active specific immune response against said lung cancer.

3. The use according to claim 1 or 2 in which said lung cancer is Non-Small Cell Lung Cancer (NSCLC).

4. The use of an immunogenic fragment of TLP comprising the following amino acid sequence: GlyProProGluValGlnAsnAlaAsn, for the manufacture of a vaccine for the preventive treatment of uro-genital cancer in mammals.

5. The use of an immunogenic fragment of TLP comprising the following amino acid sequence: GlyProProGluValGlnAsnAlaAsn, for the manufacture of an immunogenic medicament for an active specific immunotherapic treatment of a uro-genital cancer in a mammal effective to produce in said mammal an active specific immune response against said cancer.

## Patentansprüche

1. Verwendung eines immunogenen TLP-Fragments, das mindestens eine der Aminosäuresequenzen enthält, die aus der Gruppe gewählt ist, welche umfasst: ArgThrAsnLysGluAlaSerIle; GlySerAlaXPheThrAsn; AsnGlnArgAsnArgAsp, für die Herstellung eines Impfstoffs für die Präventivbehandlung von Lungenkrebs bei Säugern, der dazu wirksam ist, in diesem Säuger eine Immunantwort gegenüber Lungenkrebs zu produzieren.

2. Verwendung eines immunogenen TLP-Fragments, das mindestens eine der Aminosäuresequenzen enthält, die aus der Gruppe gewählt ist, die umfasst: ArgThrAsnLysGluAlaSerIle; GlySerAlaXPheThrAsn; AsnGlnArgAsnArgAsp, für die Herstellung eines immunogenen Medikaments für eine aktive spezifische immuntherapeutische Behandlung von Lungenkrebs in einem Säuger, das dazu wirksam ist, in dem Säuger eine aktive spezifische Immunantwort gegenüber Lungenkrebs zu produzieren.

3. Verwendung nach Anspruch 1 oder 2, bei der der Lungenkrebs ein Lungenkrebs mit nichtkleinen Zellen (NSCLC = Non-Small Cell Lung Cancer) ist.

4. Verwendung eines immunogenen TLP-Fragments, das folgende Aminosäuresequenz aufweist: GlyProProGluValGlnAsnAlaAsn, für die Herstellung eines Impfstoffs für die Präventivbehandlung von Urogenitalkrebs bei Säugern.

5. Verwendung eines immunogenen TLP-Fragments, das folgende Aminosäuresequenz aufweist: GlyProProGluValGlnAsnAlaAsn, für die Herstellung eines immunogenen Medikaments für eine aktive spezifische immuntherapeutische Behandlung eines Urogenitalkrebses in einem Säuger, das dafür wirksam ist, in dem Säuger eine aktive spezifische Immunantwort gegenüber diesem Krebs zu produzieren.

## Revendications

1. Utilisation d'un fragment immunogénique du complexe TLP, comprenant au moins l'une des séquences d'acides aminés choisies dans l'ensemble comprenant : ArgThrAsnLysGluAlaSerIle GlySerAlaXPheThrAsn AsnGlnArgAsnArgAsp pour la fabrication d'un vaccin destiné au traitement préventif du cancer du poumon chez des mammifères, efficace pour produire chez ledit mammifère une réponse immune contre ledit cancer du poumon.

2. Utilisation d'un fragment immunogénique du complexe TLP, comprenant au moins l'une des séquences d'acides aminés choisies dans l'ensemble comprenant : ArgThrAsnLysGluAlaSerIle GlySerAlaXPheThrAsn AsnGlnArgAsnArgAsp pour la fabrication d'un médicament immunogénique destiné à un traitement immunothérapeutique spécifique actif du cancer du poumon chez un mammifère, efficace pour produire chez ledit mammifère une réponse immune spécifique active contre ledit cancer du poumon.

3. Utilisation selon la revendication 1 ou 2, dans laquelle ledit cancer du poumon est un cancer du poumon non à petites cellules (NSCLC).

4. Utilisation d'un fragment immunogénique d'un complexe TLP, comprenant la séquence d'acides aminés suivante : GlyProProGluValGlnAsnAlaAsn pour la fabrication d'un vaccin destiné au traitement préventif du cancer uro-génital chez les mammifères.

5. Utilisation d'un fragment immunogénique d'un complexe TLP, comprenant la séquence d'acides aminés suivante : GlyProProGluValGlnAsnAlaAsn pour la fabrication d'un médicament immunogénique destiné à un traitement immunothérapique spécifique actif d'un cancer uro-génital chez un mammifère, efficace pour produire chez ledit mammifère une réponse immune spécifique active contre ledit cancer.
